# EUROPEAN PATENT SPECIFICATION

(11) **EP 2 349 160 B1**
(45) Date of publication and mention of the grant of the patent: **17.12.2014**
(21) Application number: 08878024.2
(22) Date of filing: 07.11.2008
(51) Int. Cl.: A61F 13/551

(54) **WRAPPER FOR ABSORBENT ARTICLE**
WICKEL FÜR EINEN SAUGFÄHIGEN ARTIKEL
EMBALLAGE POUR ARTICLE ABSORBANT

(43) Date of publication of application: 03.08.2011
(73) Proprietor: SCA Hygiene Products AB, 405 03 Göteborg (SE)
(72) Inventor: LAKSO, Elisabeth, S-444 45 Stenungsund (SE); STRIDFELDT, Chatrine, S-436 58 Hovås (SE)
(74) Representative: Zacco Sweden AB
(86) International application number: PCT/SE2008/051282
(87) International publication number: WO 2010/053417

(56) References cited:
- WO-A1-98/25562
- WO-A1-2004/060420
- WO-A1-2005/120412
- WO-A1-2005/120414
- WO-A1-2005/120414
- WO-A1-2007/050928
- WO-A1-2007/073270
- WO-A1-2008/120112
- WO-A1-2008/120112
- WO-A2-97/25955
- WO-A2-2006/101729
- WO-A2-2007/109128
- US-A1- 2006 135 927

## Description

### TECHNICAL FIELD

The invention pertains to a wrapper for an absorbent hygiene article comprising a sheet of flexible wrapping material, the sheet having a first surface being arranged to face the absorbent article and a second surface being arranged to be facing away from the absorbent article. The wrapper is adapted for use as a disposal wrap for the absorbent hygiene article.

### BACKGROUND

One problem encountered by users of hygienic absorbent articles such as feminine pads, tampons, incontinence protectors, sanitary shields, but also diapers for adults and infants, etc. is that the articles may give off an unpleasant and embarrassing odour after having been used. Sometimes, if there is no thrash-bin available, it is not possible to immediately dispose of an article after use which means that the user or a caregiver has to carry the used article until it can be thrown away. It is also desirable to reduce bad odours from used articles which are disposed of in public restrooms.

One solution to the problem would be to provide the absorbent articles with odour control agents, such as perfumes. However, many users prefer the articles to be free of added chemicals as there may be problems with allergies. Another reason for users to be hesitant to the use of scents in an absorbent article is that the user prefers to be free to choose a personal scent. WO 2005/120414 discloses a wrapper for absorbent articles, comprising a coating containing an odour control agent.

Accordingly, there is a need for a means of controlling bad odours in discarded absorbent articles.

### DISCLOSURE OF INVENTION

In accordance with the invention there is provided a wrapper as defined in claim 1.

The wrapper material can be any suitable, preferably flexible packaging material and may comprise a plastic film, a nonwoven material, a woven material, a foam material, paper or a laminate of layers of the same or different materials. The wrapper is preferably water and odour impermeable and may have a water impermeable or water repellent coating if comprising a permeable material such as paper or nonwoven. The wrapper material may further have a release coating on the inner surface, which is the surface that will be facing the wrapped absorbent article at least before use of the article. The release coating serves to protect an adhesive fixation means on the absorbent article before use of the article. The release coating may also provide water resistance or impermeability in paper and nonwovens wrapper materials.

The wrapper has at least a portion that is stretchable or elastically stretchable, as defined herein. The stretchable portion must at least have the same extent in the plane of the wrapper as the extent of the polymeric coating containing the odour control agent. The wrapper material may be stretchable over its full area by being made of a material that is inherently stretchable or that has been rendered stretchable for instance by ring-rolling, corrugating, creping, heat treatment, or by some other activation method. Alternatively, the wrapper is only stretchable within a limited portion that has been rendered stretchable or that consists of a separate stretchable piece of material.

The stretchable portion of the wrapper may be elastically stretchable which means that it will at least to some degree return to its unstretched dimensions after having been stretched.

The coating may be in the form of an adhesive coating which is non-tacky when the wrapper is in a non-stretched state and which becomes tacky after the wrapper and the coating have been stretched to the stretched state. In this embodiment of the invention, the stretched adhesive coating constitutes an adhesive disposal means that can be used to fasten the wrapper as a disposal-wrap around a used absorbent article.

The wrapper has at least one pleat such as a Z-fold or a U-fold and the coating is applied inside the pleat and is in a non-active state shielded from exposure by the pleat when the wrapper in the non-stretched state and in an active state when the wrapper has been stretched and the at least one pleat is unfolded and the coating is exposed.

The odour control agent may be arranged in microcapsules embedded in the coating.

The wrapper according to the invention is preferably a wrapper for a single hygienic absorbent article such as a sanitary napkin, a panty liner, an incontinence protector or a tampon.

The wrapper may be equally stretchable in one or more directions over its full planar extension. Alternatively, the wrapper may be made from a material that is only stretchable in a portion of the wrapper or may be made from separate materials having different stretching properties. As an example, the wrapper may be made from a non-stretchable material having a stretchable material attached along an edge portion of the non-stretchable material and carrying the polymeric coating containing the odour control agent.

Suitable materials are polymeric films and laminates comprising polypropylene, polyethylene, polyester, polyurethane, etc. It is also possible to use nonwoven materials and paper either as sole wrapper materials or in laminates. The wrapper materials are preferably chosen among those based on renewable or recycled raw materials. Blends and combinations of materials from different sources are also contemplated for the wrapper according to the invention. Examples of synthetic polymeric materials derived from renewable resources are found in WO 2007/109128. These materials are specifically not made from petroleum.

### DEFINITIONS:

Odour control agents as used herein include scents, odour absorbers, odour adsorbers, odour reducing agents that break down odorous compounds to non-odorous compounds and odour masking agents. Suitable scents and odour masking agents are perfumes or other pleasant fragrances such as vanilla, lemon, apple, cinnamon, etc. Odour absorbers or adsorbers can be cyklodextrin, zeolites, citric acid, etc. Odour reducing agents may be lactobacillus spores.

As used herein, when an odour control agent is in an active state it is exposed to the environment so that perfume or other fragrances are released and so that odour absorbers or adsorbers and odour reducing agents can act on odorous molecules.

In the non-active state, the odour control agent is shielded by being embedded in a polymeric coating composition which may be an adhesive or by being enclosed and shielded in a pleat in the wrapper. When the odour control agent is embedded in a coating composition, it can be a component held in a polymeric matrix formed by the composition or it can be in the form of microcapsules contained in the composition.

Activation takes place by stretching the wrapper to unfold the pleat and uncover the odour control agent that is contained in the pleat.

A stretchable material as used herein is a material that can be stretched to an extended state by material deformation or by unfolding of pleats or corrugations in the material. Materials that are stretchable without being elastic retain their stretched extension when stretching ceases. An example of a non-elastic stretchable material is a creped paper. An elastically stretchable material will return to a less extended state when the stretching forces are removed. A stretchable material can be stretchable in one or more directions. For a sheet material that is introduced in a production process in the form of a continuously running web, the material would typically be stretchable in a direction perpendicular to the machine direction MD, i.e. in the cross machine direction CD.

Tack is the ability of adhesive agents to form bonds instantaneously upon contact with a material surface. In the context of the invention, a tacky coating is a coating that can be used as an adhesive disposal means. The tacky coating will form a bond with the wrapper material when pressed against a surface of the wrapper and can be used to keep the wrapper in a folded configuration around a soiled absorbent article.

Non-tacky materials are materials that will not bond to the wrapper surface when being pressed against the wrapper surface.

A sanitary napkin, as used herein, is an absorbent article shaped and sized to be worn in the crotch portion of a wearer's underpants and is primarily intended for blood absorption.

Panty liners are generally smaller than sanitary napkins with lesser absorption capacity and are suitable for use between menstrual periods and for absorption of smaller amounts of vaginal discharge and urine.

As used herein an incontinence protector is an article similar in size to a sanitary napkin and shaped and configured to be worn inside a user's ordinary underwear. An incontinence protector is primarily intended for urine absorption, although for fertile women, it may serve as a combined protection against urine and blood leakage.

Tampons are usually rod-shaped absorbent articles that are inserted into the vagina where they absorb menstrual discharge.

Diapers are used by infants and adults and are in the form of absorbent pants that are pre-fastened into the pant-shape or that are fastened into the pant-shape when being put on a user.

### BRIEF DESCRIPTION OF DRAWINGS

The invention will in the following be described in greater detail with reference to the appended drawings, wherein
- Fig. 1: shows a packaging wrapper in a non-stretched state
- Fig. 2: shows the packaging wrapper in Fig. 1 in a stretched state,
- Fig. 3: shows a packaging wrapper according to the invention,
- Fig. 4: shows the packaging wrapper in Fig. 3 in a stretched state,
- Fig. 5: shows a section taken along the line V-V in Fig. 3,
- Fig. 6: shows a packaging wrapper formed into a package for a sanitary napkin before use of the napkin,
- Fig. 7: shows the wrapper in Fig. 6 when opened and before removal of the unused sanitary napkin from the wrapper,
- Fig. 8: shows the wrapper in Figs. 6 and 7 used as a disposal wrap for a soiled sanitary napkin,
- Fig. 9: shows a packaging wrapper according to the invention with an alternative arrangement of an odour control agent,
- Fig. 10: shows the packaging wrapper in Fig. 9 in a stretched state,
- Fig. 11: shows a section taken along the line IX-IX in Fig. 9,
- Fig. 12: shows a packaging wrapper having grip tabs,
- Fig. 13: shows the packaging wrapper in Fig. 12 in a stretched state,
- Fig. 14: shows a packaging wrapper in the form of a bag,
- Fig. 15: shows the packaging wrapper in Fig. 14 in a stretched state; and
- Fig. 16: shows the packaging wrapper in Figs. 14 and 15 ready for disposal and containing a used article.

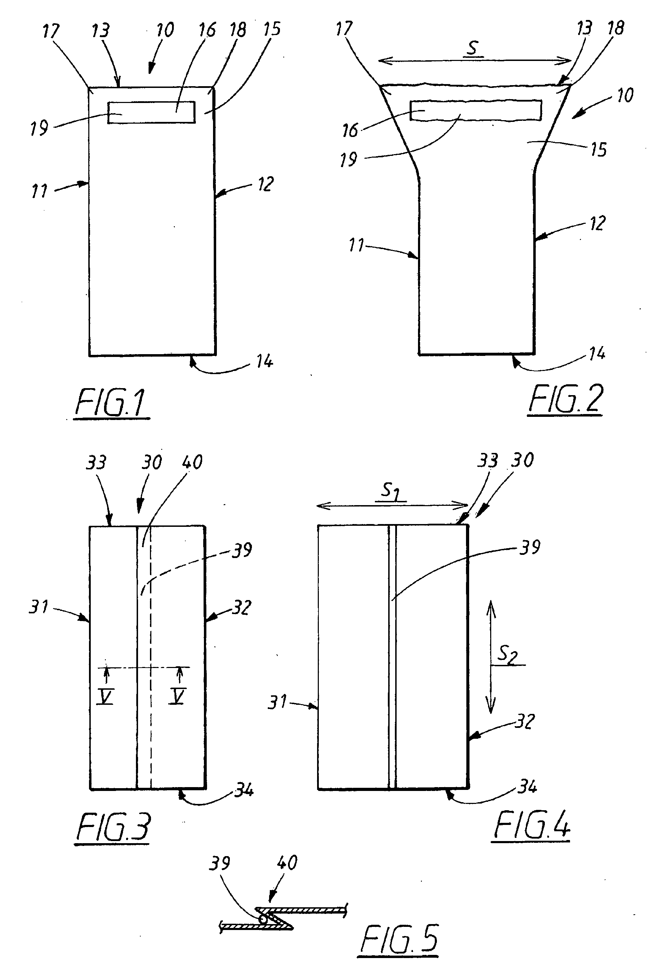

Figures 1, 2, 6-8 and 12-16 show embodiments which are not part of the invention.

The packaging wrapper 10 in Figs. 1 and 2 is in the form of a rectangular sheet of material having two longitudinal side edges, 11, 12 and two transverse end edges 13,14. The wrapper material can be any suitable preferably flexible packaging material and may comprise a plastic film, a nonwoven material, a woven material, a foam material, paper or a laminate of layers of the same or different materials. Suitable stretchable paper materials may be creped, corrugated, pleated or micropleated materials. The wrapper is preferably water and odour impermeable and may have a water impermeable or water repellent coating if comprising a permeable material such as paper or nonwoven. The wrapper material may further have a release coating on the inner surface, which is the surface that will be facing the wrapped absorbent article at least before use of the article. The release coating serves to protect an adhesive fixation means on the absorbent article before use of the article. The release coating may also provide water resistance or impermeability in paper and nonwoven wrapper materials.

The wrapper 10 has at least a portion 15 that is stretchable or elastically stretchable. The wrapper material may be stretchable over its full area by being made of a material that is inherently stretchable or that has been rendered stretchable for instance by ring-rolling, corrugating, creping, heat treatment, or by some other activation method. Alternatively, the wrapper is only stretchable within a limited portion that has been rendered stretchable or that consists of a separate stretchable piece of material.

A polymer coating 16 is applied in the form of a continuous string or band along a first end edge 13 of the wrapper within a portion 15 of the wrapper 10 that is stretchable. The polymer coating 16 contains an odour control agent 19. The odour control agent 19 may be chosen among scents, odour absorbers, odour adsorbers, odour reducing agents that break down odorous compounds to non-odorous compounds and odour masking agents. Suitable scents and odour masking agents are perfumes or other pleasant fragrances such as vanilla, lemon, apple, cinnamon, etc. Odour absorbers or adsorbers can be cyklodextrin, zeolites, citric acid, etc. Odour reducing agents may be lactobacillus spores.

The odour control agent 19 may be a component of the polymer coating that is held in a polymer matrix formed by the polymer composition or it can be in the form of microcapsules contained in the polymer composition.

In the embodiment shown in Figs. 1 and 2, the wrapper 10 is stretchable in a direction S that is parallel to the first end edge 13 and to the polymer coating 16. The odour control agent is activated by gripping the corners 17, 18 of the wrapper 10 at each side of the polymer coating 16 and stretching the wrapper material together with the coating 16 in the stretch direction S as shown in Fig 2. Stretching of the polymer coating 16 acts to open up the polymer matrix and release an odour control agent entrapped therein. Stretching will also mechanically break microcapsules containing odour control agent and allow the odour control agent to escape from the polymer coating 19.

The polymer coating 16 may be an adhesive coating which is non-tacky when the wrapper is in a non-stretched state and which becomes tacky upon stretching of the polymer coating and the wrapper 10. The stretched adhesive coating may then serve as an adhesive disposal means that can be used to secure the wrapper as a disposal-wrap around a used absorbent article.

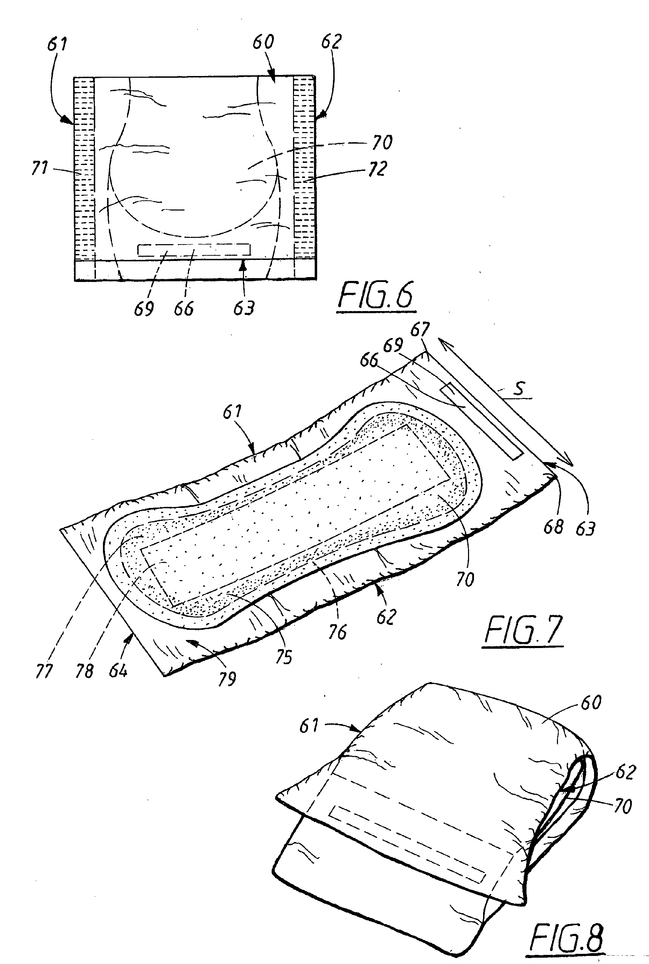

The wrapper 10 may be a packaging wrapper for an absorbent article such as a sanitary napkin, a panty liner, an incontinence shield or a tampon and will then be a protection against contamination of the unused absorbent article, for instance as shown in Fig. 6. As is conventional in the art, the packaging wrapper 10 may be provided with a release coating on the surface of the wrapper that will contact the backing layer of the absorbent article when the article is contained by the wrapper. The release coating is arranged to be in contact with any fastening adhesive on the backing layer of the absorbent article so that the packaging wrapper also constitutes a releasable protective sheet for the fastening adhesive.

The packaging wrapper 30 shown in Figs., 4 and 5, differs from that in Figs. 1 and 2 in that before stretching of the wrapper 10, the odour control agent 39 is arranged inside a Z-fold 40 as shown in Fig. 5. The Z-fold 40 is arranged in the length direction of the wrapper 30, parallel with the longitudinal side edges 31,32. Before stretching of the wrapper, the odour control agent 39 is shielded from exposure to the environment by the Z-fold. When the user pulls apart the side edges 31, 32 in a stretch direction *S₁* perpendicular to the Z-fold as shown in Fig. 4, the Z-fold becomes unfolded and the odour control agent 39 becomes exposed. In order to ensure full containment of the odour control agent 39 within the Z-fold, the fold may be sealed to completely enclose the odour control agent 39. This is normally not necessary if the odour control agent is in an immobilised form, such as in a polymeric coating.

The odour control agent 39 may be applied to the wrapper 30 in any suitable way such as by coating, printing or spraying. Preferably, the same type of coatings as in the embodiment in Figs 1 and 2 may be used. The wrapper 30 may be stretchable or elastically stretchable in a second direction *S₂* corresponding to the longitudinal direction of the wrapper 30. The odour control agent 39 may then be additionally activated by stretching in the second direction *S₂*, analogous to the activation of the odour control agent in the Figs. 1 and 2 embodiment. Activation by stretching of a coating containing the odour control agent requires the coating to be applied as a continuous string or band in the direction of stretch *S₂*. When the only release mechanism is opening up of a shielding Z-fold 39 in the wrapper 10, no particular concern has to be applied to the form of the odour control coating. Accordingly, in that case the odour control coating may be applied intermittently and in any suitable pattern.

In order to be able to secure the wrapper around a used absorbent article, the wrapper in Figs. 3-5 may in a conventional manner be provided with a fixation means such as a tape tab, an adhesive coating or a mechanical fastening arrangement such as a hook-and-loop securement means.

Fig. 6 shows a packaging wrapper 60 enclosing a packaged sanitary napkin 70. A packaging wrapper 60 of the kind shown in Figs. 6 and 7 may, of course, be used in the same manner as described in the following for packaging other disposable absorbent articles such as panty liners, incontinence shields or the like. The packaging wrapper is of the kind shown in Figs. 1 and 2. The packaging wrapper 60 has been tri-folded together with the sanitary napkin 70 and has been sealed along the open side edges 61,62 to form edge seals 71, 72. The edge seals 71, 72 may be formed by means of adhesive, by thermowelding or by ultrasonic welding. The edge seals 71, 72 are breakable seals which means that the packaging wrapper 60 can be opened up without unduly tearing and destroying the wrapper material. Breakable seals may be formed by weakening the material to create a preferential tearing line or by making the seals pealable so that the layers in the seal can be pealed apart when opening the packaging wrapper.

Fig. 7 shows the packaging wrapper 60 of Fig. 6 after it has been opened by breaking the edge seals 71, 72 and unfolding the tri-folded sanitary napkin 70 together with the packaging wrapper 60. The sanitary napkin 70 is shown from the side which is intended to be worn in contact with the user's body. The sanitary napkin has a liquid permeable topsheet 75, a liquid impermeable backing 76 and an absorbent core 77 enclosed between the topsheet 75 and the backing 76. A coating of a panty-fastening adhesive 78 is applied to the backing 77. The wrapper 60 is of the kind having a release coating on the surface 79 which is in contact with the backing 76 so that the sanitary napkin 70 can be released from the wrapper 60 and applied in a pair of underpants using the panty-fastening adhesive to attach the sanitary napkin 70 to the panty crotch.

An adhesive coating 66 containing an odour control agent 69 is arranged along a first end edge 63 of the packaging wrapper 60. The adhesive coating may be tacky in the non-stretched state or may be of the kind that requires stretching in order to activate the tack.

After removing a soiled sanitary napkin from the underpants and replacing it with a fresh napkin taken out of a packaging wrapper such as that shown in Figs. 6 and 7, the user may use the packaging wrapper 60 as a disposal wrap for the soiled sanitary napkin.

The user starts with stretching the wrapper 60 by gripping the corners 67,68 at each side of the adhesive coating 66 and stretching the wrapper 60 and the adhesive coating 66 in the stretch direction S. This will release the odour control agent 69 and activate the tack of an activatable adhesive.

The soiled napkin is subsequently folded or rolled into the wrapper 60 and the adhesive coating 66 is used to secure the wrapper 60 around the wrapped-up sanitary napkin 70.

An absorbent article such as a sanitary napkin, a panty shield, an incontinence protector or the like may similarly be packaged in a wrapper 30 of the kind shown in Figs. 3-5 which may subsequently be used as a disposal wrap for a soiled article. The wrappers 10, 30 in Figs. 1-5 may, of course also serve as disposal wraps for tampons or other personal hygiene products that need deodorisation after use.

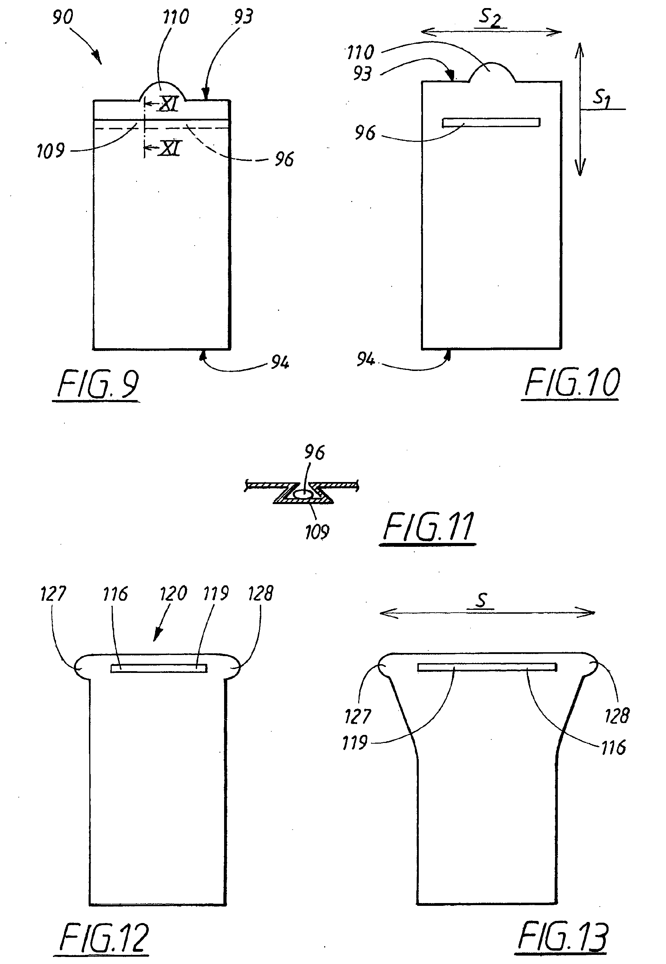

Figs. 9 and 10 illustrate two additional features that may be provided on a wrapper in accordance with the invention. The wrapper 90 in Figs. 9 and 10 has a U-fold 109 enclosing an odour control agent 96. The U-fold is arranged along a first end edge 93 of the wrapper and is opened up to reveal the odour control agent 96 by stretching of the wrapper in a stretch direction *S₁* parallel with the side edges 91, 92 of the wrapper 90. In order to facilitate stretching of the wrapper 90, a pull tab 110 is arranged centrally on the first end edge 93. A similar pull tab may be arranged extending from the opposite end edge 94, if desired. The pull tab 110 may simply be an extension of the packaging wrapper 90 itself or may be a separate component, attached to the packaging wrapper. The pull tab 110 may be made stiffer than the wrapper material by selecting a stiffer material for the pull tab or by reinforcing the pull tab with an additional layer of material. The pull tab may further be embossed or provided with perforations to increase friction and grippability. The pull tab may be a tape tab carrying a fastening adhesive that can be exposed by removing a releasable protecting layer so that the pull tab can be used as a securing means when the wrapper is used to dispose of a soiled absorbent article.

The odour control agent 96 may be completely activated by merely unfolding the U-fold 109 or may require further activation by stretching. In the latter case, the wrapper and a coating containing the odour control agent must be stretchable in a second direction *S₂* parallel to the first end edge 93.

Fig. 11 shows a section through the U-fold in Fig. 9. The odour control agent 96 is shown to be completely shielded within the U-fold 109.

Figs. 12 and 13 show a wrapper 120 of the kind shown in Figs. 1, 2 and 6-8 but with pull tabs 127, 128 arranged at the corners of the packaging wrapper at each end of the polymer coating 116 containing the odour control agent 119. The pull tabs 127, 128 may be of the same construction as the pull tab 110 in Figs. 9 and 10. The pull tabs 127, 128 facilitate stretching of the wrapper in a stretch direction S parallel with the polymer coating 116.

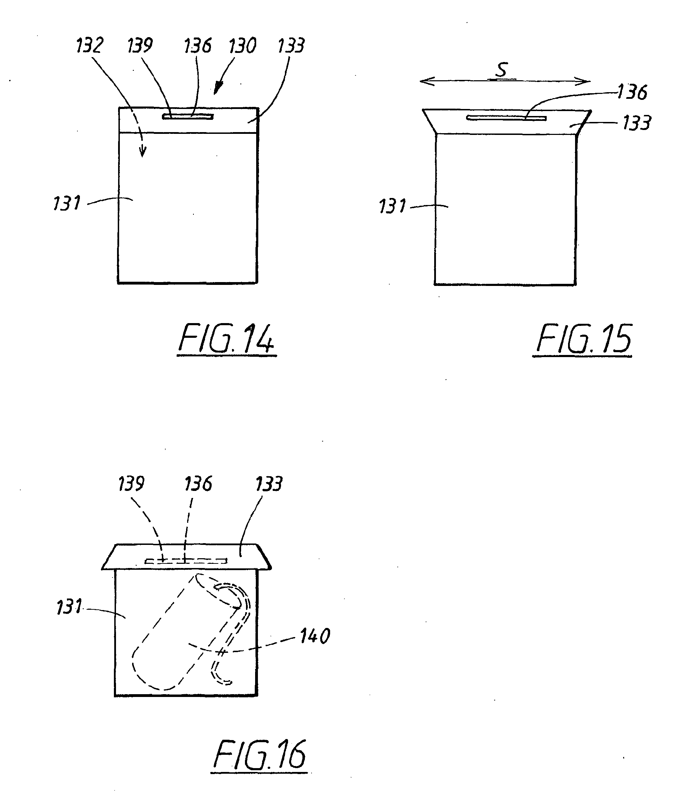

Figs. 14-16 show an embodiment of the invention where the wrapper 130 is a bag into which an absorbent article can be put for disposal. The materials for the bag may be the same as for the previously described wrappers. The wrapper bag 130 has a container portion 131 for containment of the absorbent article, the container portion 131 being closed at three sides and having an opening and a flap portion 132 extending from an edge of the opening 132. The flap portion 132 carries a polymeric coating, as described in relation to Figs. 1 and 2 and the flap portion and the polymeric coating 136 are arranged to be stretchable in a direction S parallel with the edge of the opening 132. Stretching of the polymeric coating 136 releases the odour control agent 139 and releases it for disposal. If the polymeric coating does not already exhibit sufficient tack in order to be able to be used as a securement means, the polymeric coating is also preferably activated by the stretching.

The adhesive activated flap portion 132 can then be folded over the opening 132 of a wrapper bag 130 containing a used absorbent article 140 and be secured on the outside of the bag to seal the absorbent article inside the wrapper bag 130. Fig. 16 shows a used tampon inside a sealed wrapper bag 130 with an activated odour control agent 139.

Disposal bags 130 such as shown in Figs. 14-16 may be used for disposal of other disposable hygiene articles such as sanitary napkins, diapers, panty liners, incontinence shield, etc.

It is also contemplated within the scope of the present invention to arrange the odour control agent within a Z-fold in the bag, as shown in Figs. 3 and 9. Pull tabs may also be provided as shown in Figs. 9 and 12.

Although the shown wrappers have a rectangular form, alternative shapes are contemplated within the scope of the invention. Accordingly, wrappers according to the invention may be of any suitable shape such as circular, oval, square or the like. Likewise, for a packaging wrapper, such as that shown in Figs. 6-8, it is not necessary that the wrapper is tri-folded. For smaller or stiffer products such as tampons, a double-folded wrapper or a rolled package may be more suitable. Hence, the manner in which the wrapper is formed into a package should not be considered limiting to the invention.

## Claims

1. A wrapper for an absorbent hygiene article comprising a sheet of flexible wrapping material the sheet having a first surface being arranged to face the absorbent article and a second surface being arranged to be facing away from the absorbent article, **characterized in that** the wrapper (30; 90; 130) has at least a portion that is stretchable in at least one direction (*S₁*, *S₂*) and has a polymeric coating (39; 69; 136) applied to the first and/or the second surface within the stretchable portion of the wrapper (30; 90; 130), the polymeric coating (39; 69; 136) containing an odour control agent (39; 96; 136), the odour control agent (39; 96; 136) having a first non-active state when the wrapper (30; 90; 130) is in a non-stretched state and having a second active state when the wrapper (30; 90; 130) is in a stretched state and that the wrapper has at least one pleat (40; 109) and the coating (39; 96; 136) is applied inside the pleat and is in a non-active state shielded from exposure by the pleat when the wrapper in the non-stretched state and in an active state when the wrapper has been stretched and the at least one pleat is unfolded and the coating is exposed.

2. A wrapper according to claim 1, wherein the wrapper comprises a plastic film, a nonwoven material, a woven material, a foam material, a corrugated, pleated, micropleated or creped paper or a laminate of layers of the same or different materials.

3. A wrapper according to claim 1 or 2, wherein the wrapper has a rectangular shape.

4. A wrapper according to claim 1, 2 or 3, wherein the stretchable portion of the wrapper (30; 90; 130) is elastically stretchable.

5. A wrapper according to claim 1, 2 or 3, wherein the polymeric coating (39; 96; 136) is an adhesive coating being non-tacky when the wrapper (30; 90; 130) is in a non-stretched state and being tacky when the wrapper (30; 90; 130) and the coating (39; 96; 136) have been stretched to the stretched state, and wherein the stretched adhesive coating (39; 96; 136) constitutes an adhesive disposal means.

6. A wrapper according to claim 1, 2 or 3, wherein the pleat is a Z-fold (40) or a U-fold (109).

7. A wrapper according to one of the preceding claims, wherein the coating contains the odour control agent in microcapsules embedded in the coating.

8. A wrapper according to any one of the preceding claims, wherein the wrapper is a wrapper for a single sanitary napkin, panty liner or incontinence protector.

9. A wrapper according to any one of claims 1-6, wherein the wrapper is a wrapper for a tampon.

10. A wrapper according to any one of the preceding claims, wherein the wrapper (130) is in the form of a bag.

11. A wrapper according to any one of the preceding claims, wherein the wrapper is provided with at least one pull tab (110; 127, 128).

## Patentansprüche

1. Hülle für einen saugfähigen Hygieneartikel, umfassend eine Bahn aus flexiblem Umhüllungsmaterial, wobei die Bahn eine erste Oberfläche, die dafür eingerichtet ist, dem saugfähigen Artikel zugewandt zu sein, und eine zweite Oberfläche, die dafür eingerichtet ist, vom saugfähigen Artikel abgewandt zu sein, aufweist, **dadurch gekennzeichnet, dass** die Hülle (30; 90; 130) zumindest einen Teil aufweist, der zumindest in einer Richtung (*S₁*, *S₂*) dehnbar ist und eine polymere Beschichtung (39; 69; 136) aufweist, welche auf der ersten und/oder zweiten Oberfläche innerhalb des dehnbaren Teils der Hülle (30; 90; 130) angeordnet ist, wobei die polymere Beschichtung (39; 69; 136) ein Geruchsbekämpfungsmittel (39; 96; 136) enthält, wobei das Geruchsbekämpfungsmittel (39; 96; 136) in einem ersten nicht-aktiven Zustand ist, wenn die Hülle (30; 90; 130) nicht gedehnt ist, und in einem zweiten aktiven Zustand ist, wenn die Hülle (30; 90; 130) gedehnt ist, und dass die Hülle zumindest eine Falte (40; 109) aufweist, und die Beschichtung (39; 96; 136) innerhalb der Falte angeordnet und in einem nicht-aktiven Zustand durch die Falte gegen Freilegung beschützt ist, wenn die Hülle nicht gedehnt ist, und in einem aktiven Zustand ist, wenn die Hülle gedehnt worden ist und die zumindest eine Falte entfaltet und die Beschichtung freigelegt ist.

2. Hülle nach Anspruch 1, wobei die Hülle eine Kunststofffolie, ein Vliesmaterial, ein gewebtes Material, ein Schaummaterial, ein gewelltes, gefaltetes, mikrogefaltetes oder gekrepptes Papier oder ein Laminat von Schichten aus demselben Material oder verschiedenen Materialien umfasst.

3. Hülle nach Anspruch 1 oder 2, wobei die Hülle eine rechteckige Form aufweist.

4. Hülle nach Anspruch 1, 2 oder 3, wobei der dehnbare Teil der Hülle (30; 90; 130) elastisch dehnbar ist.

5. Hülle nach Anspruch 1, 2 oder 3, wobei die polymere Beschichtung (39; 96; 136) eine klebende Beschichtung ist, die nicht haftbar ist, wenn die Hülle (30; 90; 130) nicht gedehnt ist, und haftbar ist, wenn die Hülle (30; 90; 130) und die Beschichtung (39; 96; 136) gedehnt worden sind, und wobei die gedehnte klebende Beschichtung (39; 96; 136) ein klebendes Entsorgungsmittel ausmacht.

6. Hülle nach Anspruch 1, 2 oder 3, wobei die Falte ein Z-förmiger Falz (40) oder ein U-förmiger Falz (109) ist.

7. Hülle nach einem der vorhergehenden Ansprüche, wobei die Beschichtung das Geruchsbekämpfungsmittel in in der Beschichtung eingelagerten Mikrokapseln enthält.

8. Hülle nach einem der vorgehenden Ansprüche, wobei die Hülle eine Hülle für eine einzelne Binde, einzelne Slipeinlage oder einen einzelnen Inkontinenzschutz ist.

9. Hülle nach einem der Ansprüche 1-6, wobei die Hülle eine Hülle für einen Tampon ist.

10. Hülle nach einem der vorgehenden Ansprüche, wobei die Hülle (130) in Form eines Beutels ist.

11. Hülle nach einem der vorgehenden Ansprüche, wobei die Hülle mit zumindest einer Abziehlasche (110; 127, 128) versehen ist.

## Revendications

1. Enveloppe pour un article d'hygiène absorbant comprenant une feuille de matériau souple d'enveloppe, la feuille ayant une première surface étant agencée de manière à faire face à l'article absorbant et une deuxième surface étant agencée de manière à être tournée à l'opposé de l'article absorbant, **caractérisée en ce que** l'enveloppe (30; 90; 130) présente au moins une portion qui est étirable dans au moins une direction (*S₁*, *S₂*) et présente un revêtement polymère (39; 69; 136) appliqué à la première et/ou la deuxième surface à l'intérieur de la portion étirable de l'enveloppe (30; 90; 130), le revêtement polymère (39; 69; 136) contenant un agent de contrôle des odeurs (39; 96; 136), l'agent de contrôle des odeurs (39; 96; 136) ayant un premier état non actif lorsque l'enveloppe (30; 90; 130) est dans un état non étiré, et ayant un deuxième état actif lorsque l'enveloppe (30; 90; 130) est dans un état étiré, et que l'enveloppe présente au moins un pli (40; 109) et le revêtement (39; 96; 136) est appliqué à l'intérieur du pli et est dans un état non actif à l'abri d'exposition par le pli lorsque l'enveloppe est dans l'état non étiré, et dans un état actif lorsque l'enveloppe a été étirée et l'au moins un pli est déplié et le revêtement est exposé.

2. Enveloppe selon la revendication 1, dans laquelle l'enveloppe comprend un film plastique, un matériau non-tissé, un matériau tissé, un matériau en mousse, un papier ondulé, plissé, micro-plissé ou crêpé ou un stratifié de couches de matériaux identiques ou différents.

3. Enveloppe selon la revendication 1 ou 2, dans laquelle l'enveloppe présente une forme rectangulaire.

4. Enveloppe selon la revendication 1, 2 ou 3, dans laquelle la portion étirable de l'enveloppe (30; 90; 130) est élastiquement étirable.

5. Enveloppe selon la revendication 1, 2 ou 3, dans laquelle le revêtement polymère (39; 96; 136) est un revêtement adhésif étant non collant lorsque l'enveloppe (30; 90; 130) est dans un état non étiré, et étant collant lorsque l'enveloppe (30; 90; 130) et le revêtement (39; 96; 136) ont été étirés à l'état étiré, et dans lequel le revêtement adhésif étiré (39; 96; 136) constitue un moyen d'élimination de l'adhésif.

6. Enveloppe selon la revendication 1, 2 ou 3, dans laquelle le pli est un pliage en Z (40) ou un pliage en U (109).

7. Enveloppe selon l'une des revendications précédentes, dans laquelle le revêtement comporte l'agent de contrôle des odeurs dans des microcapsules incorporées dans le revêtement.

8. Enveloppe selon l'une quelconque des revendications précédentes, dans laquelle l'enveloppe est une enveloppe pour une seule serviette hygiénique, un seul protège-slip ou une seule protection contre l'incontinence.

9. Enveloppe selon l'une quelconque des revendications 1 à 6, dans laquelle l'enveloppe est une enveloppe pour un tampon.

10. Enveloppe selon l'une quelconque des revendications précédentes, dans laquelle l'enveloppe (130) est dans la forme d'un sac.

11. Enveloppe selon l'une quelconque des revendications précédentes, dans laquelle l'enveloppe est pourvue d'au moins une languette de traction (110; 127, 128).
